# EUROPÄISCHE PATENTANMELDUNG

(11) **EP 2 570 810 A1**
(43) Veröffentlichungstag der Anmeldung: **20.03.2013**
(21) Anmeldenummer: 12185079.6
(22) Anmeldetag: 19.09.2012
(51) Int. Cl.: G01N 33/543, G01N 1/22

(54) **Verfahren zur Untersuchung von Luft auf das Vorhandensein von biologischen Substanzen mittels entfernbarer Multi-well Streifen auf denen direkt ein immunologischer Nachweis erfolgt**

(30) Priorität: 19.09.2011 DE 102011053758
(71) Anmelder: BMA Labor Gbr, 44799 Bochum (DE)
(72) Erfinder: Stephan, Ute, Dr., 44807 Bochum (DE); Putz, Stephanie, 44894 Bochum (DE)
(74) Vertreter: Vogel, Andreas

(57) **Zusammenfassung**

Die Erfindung betrifft ein Verfahren zur Untersuchung von Luft (5) auf das Vorhandensein von biologischen Substanzen, wobei eine Sammelvorrichtung (6) mit mindestens einem Probenträger (1) vorgesehen ist, die Luft (5) durch die Sammelvorrichtung (6) geleitet wird und somit eine definierte Zeit lang ein Sammelvorgang durchgeführt wird, bei dem am Probenträger (1) die biologischen Substanzen gesammelt werden, und anschließend die biologischen Substanzen durch mindestens ein immunologisches Nachweisverfahren bestimmt werden.

Erfindungsgemäß ist vorgesehen, dass nach dem Sammelvorgang der Probenträger (1) aus der Sammelvorrichtung (6) entnommen wird und in dem Probenträger (1) das immunologische Nachweisverfahren durchgeführt wird.

## Beschreibung

Die Erfindung betrifft ein Verfahren zur Untersuchung von Luft auf das Vorhandensein von biologischen Substanzen, wobei eine Sammelvorrichtung mit mindestens einem Probenträger vorgesehen ist, die Luft durch die Sammelvorrichtung geleitet wird und somit eine definierte Zeit lang ein Sammelvorgang durchgeführt wird, bei dem am Probenträger die biologischen Substanzen gesammelt werden und anschließend die biologischen Substanzen durch mindestens ein immunologisches Nachweisverfahren bestimmt werden. Ferner bezieht sich die Erfindung auf eine Sammelvorrichtung zum Sammeln der biologischen Substanzen aus der Luft, sowie auf ein immunologisches Verfahren zum Nachweis der gesammelten biologischen Substanzen.

In der US 2008/0184515 A1 ist sowohl ein Verfahren zur Untersuchung von Luft auf das Vorhandensein von Staub und einzelnen Partikeln, wie z. B. biologischen Substanzen, als auch eine Sammelvorrichtung mit Adapter offenbart. Diese Sammelvorrichtung wird in Innenräumen verwendet und dient dem Sammeln von insbesondere Allergenen. Die Sammelvorrichtung besteht dabei aus einer Düse, einem Probenträger in Form eines Filters, sowie einem Adapter, der es ermöglicht die Düse auf verschiedene Saugpumpen aufzumontieren. Ein Luftstrom wird dabei durch den Filter geleitet und dieser sammelt so alle Partikel größer als die Filterporen. Nach dem Sammelvorgang werden der Staub und die Partikel insbesondere die Antigene, insbesondere die Allergene, in einem Nachweisverfahren, bevorzugt einem ELISA (Enzyme Linked Immunosorbent Assay) analysiert.

In der EP 1 411 229 A1 ist sowohl ein Verfahren zur automatischen Untersuchung von Proteinen aus biologischen Substanzen, als auch die Vorrichtung, in der das automatisierte Verfahren durchgeführt wird, offenbart. Das Verfahren und die Vorrichtung werden dabei dafür eingesetzt jeweils ein spezifisches Protein aus biologischen Proben nachzuweisen. Der Nachweis in dieser Schrift erfolgt bevorzugt über einen ELISA.

Nachteiligerweise hat sich gezeigt, dass das Sammeln biologischer Substanzen auf Filtern einen hohen Zeit- und Kostenaufwand erfordert und das Herauslösen der biologischen Substanzen erschwert ist. Ferner ist das Herauslösen aus einem Filter mit hohen Verlusten biologischer Substanzen verbunden, die eine anschließende, verlässliche Quantifizierung der gesammelten Substanzen in einem messbaren Bereich erschweren. Weiterhin sind durch den Gebrauch von Filtern weitere Arbeitsschritte notwendig, die mit höheren Kosten verbunden sind. Ebenfalls höhere Kosten und einen gesteigerten Arbeitsaufwand verursacht ein Nachweis lediglich einzelner biologischer Substanzen.

Die Aufgabe der vorliegenden Erfindung ist es, die oben genannten Nachteile zu vermeiden, insbesondere ein Verfahren, eine Sammelvorrichtung und ein immunologisches Verfahren bereitzustellen, wodurch die Untersuchung auf das Vorhandensein von biologischen Substanzen vereinfacht ist. Ferner soll sowohl ein schnelleres, bedingt durch weniger Arbeitsschritte, und auch ein kostengünstiges immunologisches Verfahren ermöglicht werden.

Zur Lösung dieser Aufgabe wird ein Verfahren mit den Merkmalen des Anspruches 1 sowie eine Sammelvorrichtung mit den Merkmalen des Anspruches 9 vorgeschlagen. In den abhängigen Ansprüchen sind bevorzugte Weiterbildungen ausgeführt. Des Weiteren wird ein immunologisches Verfahren mit den Merkmalen des Anspruches 13 vorgeschlagen, das ebenfalls die oben genannte Aufgabe löst.

Dazu ist erfindungsgemäß vorgesehen, dass der Probenträger nach dem Sammelvorgang aus der Sammelvorrichtung entnommen wird und in dem Probenträger das immunologische Nachweisverfahren durchgeführt wird. Der wesentliche Kern der Erfindung ist, dass der Probenträger, in dem die biologischen Substanzen gesammelt werden, genutzt wird, um im selben Gefäß das immunologische Verfahren durchzuführen und den Nachweis biologischer Substanzen zu erbringen. Insbesondere wird daher ein nahezu verlustfreies Sammeln biologischer Substanzen ermöglicht, da die biologischen Substanzen nicht erst aus einem Probenträger herausgelöst werden, um anschließend in ein Gefäß, in welchem der immunologische Nachweis stattfindet, überführt werden müssen. Dadurch ergeben sich Zeit - und Kostenersparnisse beim Sammelvorgang und für die benötigten Materialien. Ebenfalls kann so nicht nur auf ein Überführen der Proben, sondern auch auf einen Auswaschvorgang der Proben aus einem Probenträger verzichtet werden. Somit können weitaus kleinere Mengen an biologischen Substanzen nachgewiesen werden, da Verluste minimiert werden können. Auch die Zeit, die von der Sammlung bis zur Durchführung des immunologischen Nachweisverfahrens aufgebracht werden muss, ist reduziert, was wiederum zu einer Kosteneinsparung führt.

Vorteilhafterweise werden in dem Verfahren zur Untersuchung biologischer Substanzen aus der Luft Proteine nachgewiesen, die insbesondere in Form von Antigenen und besonders bevorzugt in Form von Allergenen detektiert werden können. Da viele Menschen allergische Reaktionen auf biologische Substanzen zeigen, sind die nachzuweisenden Allergene von Milben und/oder Tieren und/oder Kakerlaken und/oder Schimmelpilzen und/oder Pollen und/oder allergische Reaktionen auf Gummi, insbesondere Latex im Hinblick auf eine gezielte Expositionsvermeidung und/oder Minimierung einer Allergenbelastung von entscheidender Bedeutung.

Weiterhin ist es denkbar, dass in dem immunologischen Nachweisverfahren zur Identifikation der biologischen Substanzen, bevorzugt der Allergene, Antikörper eingesetzt werden, welche sich die natürliche Allergen/Antigen-Anitikörper-Erkennung zu Nutze machen. Antikörper sind Proteine, die zur Erkennung von Antigenen, insbesondere Allergenen gebildet werden. In der Regel dient u. a. die Bildung von Antikörpern im Rahmen einer Immunreaktion der Abwehr bzw. dem Schutz des Organismus gegenüber Krankheiten. Im Falle einer Überempfindlichkeitsreaktion bzw. Allergie kommt es jedoch zu einer überschießenden Immunreaktion, die Krankheitssymptome auslösen kann und somit für den betroffenen Orgnismus negative Auswirkungen haben kann. Bei dem Einsatz von Antikörpern im immunologischen Nachweisverfahren ist es von Vorteil das Antikörper sehr spezifisch sind und nur passgenaue Allergene nachweisen.

Vorteilhafterweise werden bei dem Sammelvorgang mindestens 100 Liter bevorzugt mindestens 1.000 Liter und besonders bevorzugt mindestens 2.000 Liter Luft gesammelt. Insgesamt werden dabei mindestens 50 Liter pro Minute und bevorzugt mindestens 100 Liter pro Minute Luft durch die Sammelvorrichtung geleitet. Somit kann ein Sammelvorgang weniger als vier Stunden, bevorzugt weniger als zwei Stunden und besonders bevorzugt weniger als eine Stunde dauern. Bei einem durchzuleitenden Luftvolumen von ungefähr 2.000 Litern und einer Sammelintensität von ungefähr 100 Litern pro Minute ist der Sammelvorgang auf 20 Minuten verkürzt. Diese Ersparnis an Zeit ermöglicht es in derselben Zeit, die andere Verfahren für eine einzelne Messung benötigen, gleich mehrere Messungen durchzuführen, was zu einem weitaus genaueren sowie schnellerem Ergebnis führt.

In einer weiteren Möglichkeit der Erfindung, wird der Sammelvorgang der Luft in Innenräumen ausgeführt. Im Gegensatz zu allergenen, biologischen Substanzen der Außenwelt kommen diese in geschlossenen Räumen häufig in weitaus höheren Konzentrationen vor, so dass mögliche gesundheitsgefährdende Grenzwerte öfter und schneller erreicht werden, als in der Außenwelt. In Innenräumen können sich biologische Substanzen mit sensibilisierendem Potential unter bestimmten Bedingungen, wie besipielsweise hoher Luftfeuchtigkeit und/oder hoher Temperatur, anreichern, so dass mögliche gesundheitsgefährdende Risiken für die Raumnutzer auftreten können. Daher ist es von großer Bedeutung Messungen in Innenräumen durchführen zu können, weil man den Substanzen dort ausgesetzt ist. Zusätzlich sind das Verfahren und auch die Sammelvorrichtung so ausgestaltet, dass das Sammeln der biologischen Substanzen ebenfalls in der Außenwelt durchgeführt werden kann. Die gesammelten Proben aus der Außenwelt dienen oftmals als Referenzwerte. Ein weiterer Vorteil von schnellen und kostengünstig durchführbaren Innenraummessungen und dazugehörigen Referenzmessungen außerhalb geschlossener Räumlichkeiten ist, dass eine genaue Lokalisation des Ursprungs der biologischen Substanzen ermöglicht werden kann. Basierend auf den Messergebnissen kann möglicherweise eine gezielte Quellensuche und Sanierung erfolgen.

In einer möglichen Ausführungsform ist der Probenträger für den Sammelvorgang filterlos ausgeführt. Der Sammelvorgang findet dabei direkt in dem Probenträger statt. Der Probenträger ist dabei undurchlässig für jegliche Art von Medien ausgestaltet, insbesondere auch für Luft und darin enthaltene biologische Substanzen. Die während des Sammelns in den Probenträger eingeleitete Luft durchdringt den Probenträger nicht, sondern entweicht in derselben Richtung, in welcher die Luft eingeströmt ist. Bedingt durch die Undurchlässigkeit des Probenträgers, können auch noch so kleine biologische Substanzen, bevorzugt kleiner als 1 µm, besonders bevorzugt kleiner als 0,5 µm und besonders bevorzugt kleiner als 0,1 µm, nicht aus dem Probenträger entweichen. Bei einem Probenträger, der einen Filter umfasst, ist die Größe der sammelbaren biologischen Substanzen deutlich eingeschränkt, wohingegen filterlose Probenträger biologische Substanzen unabhängig von ihrer Größe sammeln können. Darüber hinaus können so möglicherweise biologische Substanzen, die in sehr geringen Konzentrationen in der Luft vorkommen, zuverlässig und ohne Überführung in ein zusätzliches Extraktionsgefäß gesammelt werden. Werden zum Sammeln hingegen Filter als Probenträger eingesetzt, so kann aufgrund der notwendigen Extraktion in einem zusätzlichen Gefäß und/oder aufgrund ungünstiger Bindungeigenschaften und/oder durch ungünstige Lösungsvolumenverhältnisse bei der Extraktion zu Verlusten bzw. zu Verdünnungen der zu untersuchenden biologischen Substanzen kommen.

Vorteilhafterweise sind die Probenträger, in denen die biologischen Substanzen gesammelt werden, als Mikroreaktionsgefäße ausgestaltet. Da biologische Substanzen in der Regel mehrfach bestimmt werden, ist es von Vorteil gleichzeitig in einem Sammelvorgang, biologische Substanzen in mehreren Mikroreaktionsgefäßen zu sammeln. Die Mikroreaktionsgefäße sind deswegen in einer vorteilhaften Ausführungsform als Mikroreaktionsgefäßstreifen ausgestaltet. Ein Streifen weist dabei mehrere, bevorzugt acht, Mikroreaktionsgefäße, die bevorzugtKavitäten aufweisen, auf, die in einer Reihe, in einer Ebenen zueinander fluchtend, angeordnet sind und die in der Regel ein Volumen von je 400 µl aufweisen. Die biologischen Substanzen können so in den Gefäßen in einem Sammelvorgang gesammelt werden. Vorteilhafterweise wird während des Sammelvorganges in ein Gefäß nahezu keine Luft und somit keine biologischen Substanzen eingeleitet, wobei dieses Gefäß bei dem anschließenden Messverfahren als Kontrollgefäß, bevorzugt als Negativkontrolle,dient. Da nahezu keine Luft und somit keine biologischen Substanzen in dieses Gefäß gelangt sind, wird es als Negativkontrolle eingesetzt. Der Vorteil einer Negativkontrolle, die denselben Bedingungen ausgesetzt ist, wie alle anderen Sammelgefäße, lässt sowohl Rückschlüsse auf die Luftprobennahme, als auch auf eine fehlerfreie Versuchsdurchführung des immunologischen Nachweisverfahrens zu. Da die erforderliche, fehlerfreie Durchführung eines immunologischen Nachweisverfahrens von vielen Faktoren, wie Temperatur und/oder Haltbarkeit der Lösung und/oder Spezifität der Antikörper abhängt, ist eine Negativkontrolle, als interne Versuchskontrolle von großem Vorteil, um keine falschen Ergebnisse zu erzielen.

In einer bevorzugten Ausführungsform erfolgt der Nachweis der gesammelten biologischen Substanzen über eine enzymatische Reaktion, bevorzugt eine enzymatische Farbreaktion. Der entstandene Farbumschlag kann anschließend über eine photometrische Messung bestimmt werden, wobei diese Messung bevorzugt bei einer optischen Dichte von 400 bis 500 nm und bevorzugt bei ungefähr 405 oder 492 nm durchgeführt wird. Enzymatische Farbreaktionen im immunologischen Nachweisverfahren dienen dabei vorteilhafterweise nicht nur der Qualifizierung, sondern auch der Quantifizierung von nachzuweisenden Antigenen. Ferner ist durch einen photometrischen Beweis bei einer bestimmten Wellenlänge eine weitere Spezifität des immunologischen Nachweisverfahrens gegeben.

Die vorliegende Erfindung wird ebenfalls durch eine Sammelvorrichtung mit den Merkmalen des Anspruches 9 ermöglicht. Erfindungsgemäß ist der Probenträger gefäßartig mit einer definierten Bindungskapazität derart ausgestaltet, dass beim Sammelvorgang biologische Substanzen zuverlässig in diesem verbleiben. Der mögliche Kern der Erfindung ist, dass die gefäßartigen Probenträger derart ausgebildet sind, dass diese eine hohe Bindungskapazität aufweisen, so dass biologische Substanzen an Gefäßwänden haften bleiben. Insbesondere wird dadurch ein verlustfreies Sammeln biologischer Substanzen ermöglicht, da die biologischen Substanzen nicht erst aus einem Probenträger herausgelöst werden, um anschließend in ein Gefäß, in welchem der immunologische Nachweis stattfindet, überführt werden müssen. Dadurch können des Weiteren Kosten für Material eingespart werden. Ebenfalls kann so nicht nur auf ein Überführen der Proben sondern auch auf einen Auswaschvorgang verzichtet werden. Somit können auch weitaus geringere Konzentrationen an biologischen Substanzen nachgewiesen werden, da kaum Verluste vorkommen. Auch die Zeit, die von der Sammlung bis zur Durchführung des immunologischen Nachweisverfahrens aufgebracht werden muss, ist minimiert, was wiederum zu einer Kosteneinsparung führt.

In einer vorteilhaften Ausführung der Erfindung sind die Probenträger in Form von Mikroreaktionsgefäßen ausgestaltet, wobei mehrere Mikroreaktionsgefäße in einer Reihe angeordnet sind. Die Mikroreaktionsgefäße sind dabei aus Kunststoff gefertigt, bevorzugt aus Polystyrol, und bilden eine zylindrische Form. In einer bevorzugten Ausführungsform der Erfindung können ferner auf die Mikroreaktionsgefäße angepasste Deckel zum Verschließen der Mikroreaktionsgefäße, bevorzugt aus Polypropylen, ausgestaltet sein. Der Vorteil einer Ausführungsform der Mikroreaktionsgefäße aus Polystyrol ist, dass dieses Material überraschenderweise eine hohe Bindungskapazität aufweist und somit die mit der Luft eingeleiteten biologischen Substanzen an den Gefäßwänden haften bleiben. Die zylindrische Ausgestaltung der Mikroreaktionsgefäße ist sowohl für den Sammelvorgang, da der Luftstrom optimal an den Gefäßwänden entlang geleitet werden kann, als auch für das anschließende immunologische Verfahren, da die Gefäße auf dieses Verfahren abgestimmt sind, von großem Vorteil. Mit den zugehörigen Deckeln können die Mikroreaktionsgefäße verschlossen werden, um die gesammelten biologischen Substanzen verlustfrei transportieren zu können. Vorteilhafterweise sind die Deckel aus Polypropylen, einem anderen Kunststoff gefertigt, der keine hohe Bindungskapazität aufweist, damit die gesammelten biologischen Substanzen nicht an den Deckeln verbleiben, wo diese erst abgelöst werden müssten, was zusätzliche Zeit und Kosten verursachen würde. Ferner können die Mikroreaktionsgefäße zusätzlich beschichtet werden, bevorzugt mit Glutaraldehyd und/oder einem entsprechenden Antikörper, um eine noch weiter erhöhte Bindungsfähigkeit zu erzeugen, damit die Menge an gesammelten biologischen Substanzen noch weiter gesteigert werden kann.

Die vorliegende Erfindung wird ebenfalls durch ein immunologisches Verfahren mit den Merkmalen des Anspruches 13 ermöglicht. Erfindungsgemäß ist vorgesehen, dass zur Erkennung der gesammelten Antigene ein Gemisch aus mindestens zwei Antikörpern zwei verschiedene Arten von biologischen Schadstoffen eingesetzt wird. Der wesentliche Kern der Erfindung ist, dass mindestens zwei verschiedene Antikörper, die Antigene von mindestens zwei verschiedenen Schimmelpilzarten und/oder Milben und/oder Tieren, insbesondere Haustieren, und/oder Kakerlaken und/oder Pollen und/oder Gummi, insbesondere Latex erkennen können. Vorteilhafterweise wird ein Nachweis mehrerer allergener Substanzen in einem Nachweisverfahren ermöglicht. Dadurch wird eine Kosten - und Zeitersparnis erreicht, weil mit einem durchgeführten Nachweis mehrere Antigene auf einmal nachgewiesen werden können.

Vorteilhafterweise wird ein Serum zur Erkennung der biologischen Substanzen eingesetzt. Das Serum ist dabei in einem immunisierten Säugetier gewonnen worden, bevorzugt in einem Nagetier und besonders bevorzugt in einem Kaninchen. Vorteilhafterweise werden so, polyklonale Antikörper gewonnen, die ein breites Erkennungsspektrum besitzen. Polyklonale Antikörper sind zur Erkennung gleich mehrerer spezifischer Stellen auf biologischen Substanzen geeignet, was die Testspezifität verringert, die Sensitivität jedoch erhöht. Besonders die Verwendung von mindestens zwei Antigenen aus mindestens zwei verschiedenen biologischen Substanzen, die zur Gewinnung des Serums eingesetzt werden, reduziert den Verbrauch an Versuchstieren und somit ebenfalls die Kosten. In einer bevorzugten Ausführungsform wird das Serum aus einem Gemisch aus Antigenen mindestens zwei verschiedener Arten hergestellt. Dabei ist ein Gemisch aus mindestens vier oder mindestens 100 verschiedenen Antigenen besonders bevorzugt. Je mehr Antigene verschiedener Arten an biologischen Schadstoffen auf einmal zur Immunisierung von Säugetieren, bevorzugt von Kaninchen, eingesetzt werden, desto weiter können die Kosten reduziert werden, da sowohl weniger Versuchstiere benötigt, als auch weniger Nachweisverfahren durchgeführt werden müssen.

In einer bevorzugten Ausführungsform sind Antikörper zur Erkennung der Schimmelpilzarten Aspergillus penicilloides und/oder Cladosporium cladosporioides im Serum zum Nachweis der Antigene eben dieser Arten enthalten. Ein Gemisch aus Antikörpern fasst die Erkennung aus einer Kombination der Antigene von Aspergillus penicilloides und Aspergillus versicolor und/oder ein Gemisch aus Aspergillus penicilloides und Penicillium chrysogenum und/oder aus Cladosporium cladosporioides und Aspergillus versicolor und/oder aus Cladosporium cladosporioides und Penicillium chrysogenum und/oder aus Aspergillus versicolor und Penicillium chrysogenum und/oder aus Aspergillus penicilloides und Cladosporium cladosporioides und Aspergillus versicolor und/oder aus Aspergillus penicilloides und Cladosporium cladosporioides und Penicillium chrysogenum und/oder aus Aspergillus penicilloides und Aspergillus versicolor und Penicillium chrysogenum und/oder aus Cladosporium cladosporioides und Aspergillus versicolor und Penicillium chrysogenum und/oder aus Cladosporium cladosporioides und Penicillium chrysogenum und Aspergillus penicilloides und/oder aus Aspergillus penicilloides und Cladosporium cladosporioides und Aspergillus versicolor und Penicillium chrysogenum. Dabei sind die Schimmelpilzarten Aspergillus penicilloides, Cladosporium cladosporioides, Penicillium chrysogenum und Aspergillus versicolor, die am häufigsten in Innenräumen vorkommenden Arten, die gleichzeitig als potentielle Risikofaktoren für die Gesundheit bei entsprechenden prädisponierten Individuen betrachtet werden.

Des Weiteren können Antikörper gegen alle weiteren Vertreter der Gattung Aspergillus und/oder Eurotium und/oder Paecilomyces und/oder Penicillium und/oder Cladosporium zur Erkennung der gesammelten Antigene herangezogen werden. Die Gattung Aspergillus umfasst dabei die Arten Aspergillus nidulans, Aspergillus ochraceus, Aspergillus candidus, Aspergillus clavatus, Aspergillus flavus var. flavus, Aspergillus niger, Aspergillus penicilloides, Aspergillus versicolor, Aspergillus ustus, Aspergillus flavus, Aspergillus sydowii, Aspergillus terreus, Aspergillus restrictus, Aspergillus fumigatus, Aspergillus tamarii. Die Gattung Eurotium umfasst dabei die Arten Eurotium chevalieri, Eurotium herbariorum, Eurotium amstelodami. Die Gattung Paecilomyces umfasst dabei die Arten Paecilomyces fumosoroseus, Paecilomyces variotii, Paecilomyces lilacinus. Und die Gattung Penicillium umfasst dabei die Arten Penicillium nalgiovense, Penicillium camemberti, Penicillium chrysogenum, Penicillium digitatum, Penicillium glabrum, Penicillium rugulosum, Penicillium roquefortii, Penicillium corylophilum, Penicillium brevicompactum, Penicillium olsonii, Penicillium glabrum, Penicillium citrinum, Penicillium corylophylum, Penicillium crustosum, Penicillium funiculosum, Penicillium variabile, Penicillium variabile 1, Penicillium verrucosum, Penicillium olsonii, Penicillium expansum.

Sämtliche Arten aus einem Gemisch aus mindestens zwei, bevorzugt aus mindestens vier und besonders bevorzugt aus mehr aus mindestens 100 Antikörpern in einer beliebigen Kombination verschiedener Arten der Gattung Aspergillus und/oder Eurotium und/oder Paecilomyces und/oder Penicillium und/oder Cladosporium können zur Herstellung eines Serums kombiniert werden. Darüber hinaus sind ebenfalls die Arten Acremonium furcatum, Chaetomium funicola, Chaetomium sp. (evtl. globosum), Cunninghamella sp., Fusarium oxysporum, Myrothecium sp. Oder Doratomyces sp., Sporothrix schenckii, Beauveria bassiana, Ascotricha chartarum, Exophiala jeanselmei, Absidia corymbifera, Acremonium strictum, Alternaria alternata, Botrytis cinerea, Byssochlamis nivea, Curvularia geniculata, Emericella nidulans, Epicoccum nigrum, Fusarium culmorum, Fusarium sporotrichioides var. sporotrichioid, Geotrichum candidum, Mucor plumbeus, Phoma glomerata, Stachybotrys chartarum, Syncephalastrum racemosum, Ulocladium chartarum, Wallemia sebi, Emericella nidulans, Scopulariopsis brevicaulis, Mucor racemosus, Syncephalastrum racemosus, Chaetomium globosum, Stachybotrys chartarum, Phoma glomerata, Geomyces pannorum, Scopulariopsis fusca, Trichothecium roseum, Candida albicans, Absidia corymbifera, Mucor plumbeus, Aureobasidium pullulans, Epicoccum nigrum, Botrytis cinerea, Geotrichum candidum, Rhizopus stolonifer, Trichurus sp. / Doratomyces sp. bevorzugte Arten zur Herstellung eines Serums geeignet.

Des Weiteren können Antikörper gegen alle weiteren Tierarten wie z. B. Vertreter der Gattung Felis, insbesondere Felis domesticus, und/oder Canis, insbesondere Canis familiaris, und/oder Rattus und/oder Mus und/oder Blattella, insbesondere Blattella germanica, zur Erkennung der gesammelten Antigene herangezogen werden. Ferner können Antikörper gegen alle weitere relevanten Pollen wie z. B. die Gattung Betula, insbesondere Betula verrucosa, und/oder Phleum, insbesondere Phleum pratense, und/oder Ambriosa, insbesondere Ambriosa atremisiifolia, zur Erkennung der gesammelten Antigene herangezogen werden. Weiterhin können Antikörper gegen alle weitere relevanten Milben wie z. B. Vertreter der Gattung Dermatophagoides, insbesondere Dermatophagoides sp., Dermatophagoides pteronyssinus, Dermatophagoides farinae, und/oder Blomia, insbesondere Blomia tropicalis, zur Erkennung der gesammelten Antigene herangezogen werden. Des Weiteren können Antikörper gegen Gummi, insbesondere Latex wie z. B. die Allergene Hev b 1 und/oder Hev b3 und/oder Hev b3 und/oder Hev b 6.02 zur Erkennung der gesammelten Antigene herangezogen werden.

Weitere Vorteile, Merkmale und Einzelheiten der Erfindung ergeben sich aus der nachfolgenden Beschreibung, in der unter Bezugnahme auf die Zeichnungen mehrere Ausführungsbeispiele der Erfindung beschrieben sind. Dabei können die in den Ansprüchen und in der Beschreibung erwähnten Merkmale jeweils einzeln für sich oder in beliebiger Kombination erfindungswesentlich sein. Es zeigen:
Fig. 1 schematische Darstellung der Probenträger,
Fig. 2 schematische Darstellung des Luftstromes im Probenträger,
Fig. 3 schematische Darstellung der Sammelvorrichtung,
Fig. 4 schematischer Ablauf des Versuchsverlaufes und
Fig. 5 schematischer Ablauf des immunologischen Nachweisverfahrens.

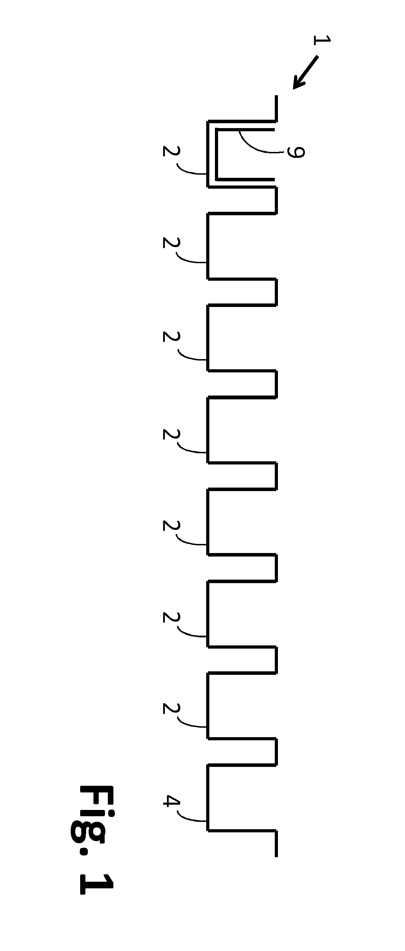

In Figur 1 ist ein Probenträger 1 gezeigt, der als ein Mikroreaktionsgefäß 2 dargestellt ist, in welchem biologische Proben aus der Luft 5 gesammelt werden können. Der Probenträger 1 ist in dem vorliegenden Ausführungsbeispiel als Mikroreaktionsgefäßstreifen 1 dargestellt, wobei mehrere, insbesondere acht Mikroreaktionsgefäße 2, in einer Ebene fluchtend, miteinander verbunden sind. Die Mikroreaktionsgefäße 2 sind dabei bevorzugt aus Kunststoff hergestellt. Kunststoff, insbesondere Polystyrol, hat dabei die Eigenschaft eine hohe Bindungskapazität aufzuweisen, damit die biologischen Substanzen an den Wänden der Mikroreaktionsgefäße 2 verbleiben. Neben der durch den Kunststoff bedingten Haftungseigenschaften, können die Mikroreaktionsgefäße 2 zusätzlich, bevorzugt mit Glutaraldehyd und/oder einem Antikörper, beschichtet 9 sein. Alle Mikroreaktionsgefäße 2 in einem Mikroreaktionsgefäßstreifen 1 weisen dabei dieselbe Beschichtung auf. Ein Mikroreaktionsgefäß 2 in einem Mikroreaktionsgefäßstreifen 1 kann als Kontrolle 4 eingesetzt werden. Die Kontrolle 4 befindet sich bevorzugt an den äußeren Enden des Mikroreaktionsgefäßstreifens 1.

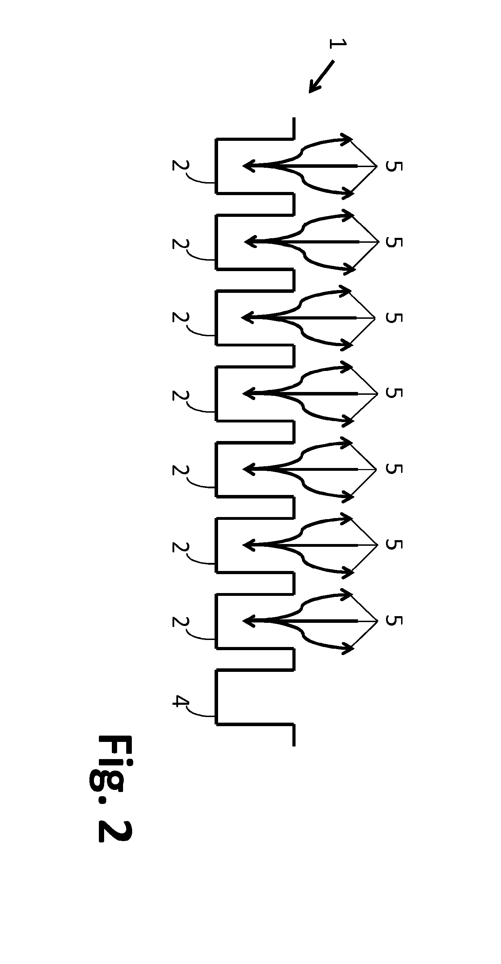

Figur 2 zeigt einen Probenträger 1 in Form eines Mikroreaktionsgefäßstreifens 1, wobei schematisch der Luftstrom 5 dargestellt ist. Der Luftstrom 5 mit den biologischen Substanzen wird dabei von oben in die Mikroreaktionsgefäße 2 eingeleitet. Da die Mikroreaktionsgefäße 2 luftundurchlässig ausgestaltet sind, wird der Luftstrom 5 zu den Seiten abgelenkt und entweicht nach oben hin aus den einzelnen Mikroreaktionsgefäßen 2. Die im Luftstrom 5 mitgeführten biologischen Substanzen bleiben dabei an den Innenflächen der Mikroreaktionsgefäße haften. Lediglich in das Kontrollgefäß 4, welches als Negativkontrolle dient, gelangt nahezu keine Luft 5.

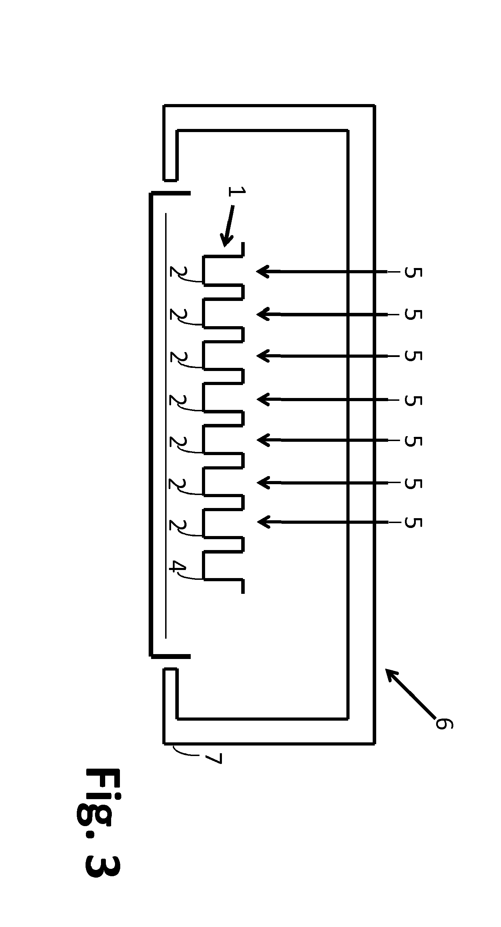

In Figur 3 ist die Sammelvorrichtung 6 zum Sammeln der biologischen Substanzen aus der Luft 5 gezeigt. Die Sammelvorrichtung 6 kann ein Gehäuse 7 aufweisen, in welches die Luft 5 eingeleitet wird, so dass ein Probenträger 1 in Form eines Mikroreaktionsgefäßstreifens 1 umschlossen wird. Die Luft 5 wird dabei so in die Mikroreaktionsgefäße 2 eingeleitet, dass sie von oben in die Öffnung der Gefäße 2 strömt und zu den Seiten abgelenkt wird und wieder nach oben hinaus aus den Mikroreaktionsgefäßen 2 entweicht. In das Mikroreaktionsgefäß 2, welches als Kontrolle 4 dient, wird nahezu keine Luft 5 und somit auch nahezu keine biologischen Substanzen eingeleitet, so dass auch nahezu keine biologischen Substanzen in diesem Mikroreaktionsgefäß 2 verbleiben.

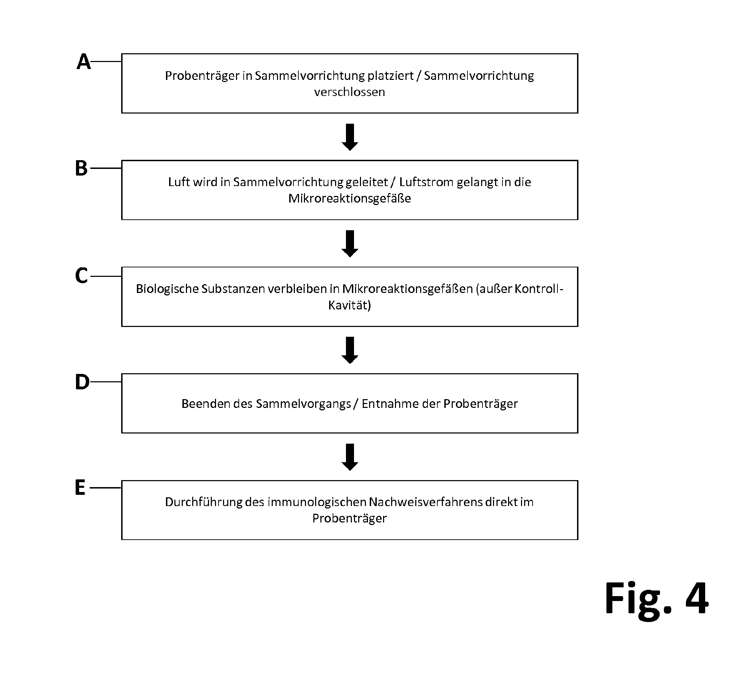

Figur 4 zeigt ein Diagramm über den Verlauf des Sammelvorganges mit anschließendem Nachweisverfahren. Im ersten Schritt (A) wird ein Probenträger 1 in Form eines Mikroreaktionsgefäßstreifens 1 in der Sammelvorrichtung 6 platziert und die Sammelvorrichtung 6 wird verschlossen. Anschließend (B) wird Luft 5 durch die Sammelvorrichtung 6 geleitet, so dass der Luftstrom 5 von oben in die einzelnen Mikroreaktionsgefäße 2 eingeleitet wird. Der Luftstrom 5 wird in dem Mikroreaktionsgefäß 2 so abgelenkt, dass er nach oben hin wieder aus diesem entweicht. Unterdessen verbleibend, bedingt durch die hohe Bindungskapazität bzw. wegen einer aufgetragenen Beschichtung 9, die biologischen Substanzen in den Mikroreaktionsgefäßen 2 (C). Lediglich in das Kontrollgefäß 4 wird nahezu keine Luft 5 eingeleitet, so dass sich auch nahezu keine biologischen Substanzen in einem Kontrollmikroreaktionsgefäß 2 befinden (C). Nachdem der Sammelvorgang beendet ist, wird der Probenträger 1 aus der Sammelvorrichtung 6 entnommen (D). Direkt im Anschluss kann in den zum Sammeln verwendeten Probenträgern 1 das immunologische Nachweisverfahren durchgeführt werden (E).

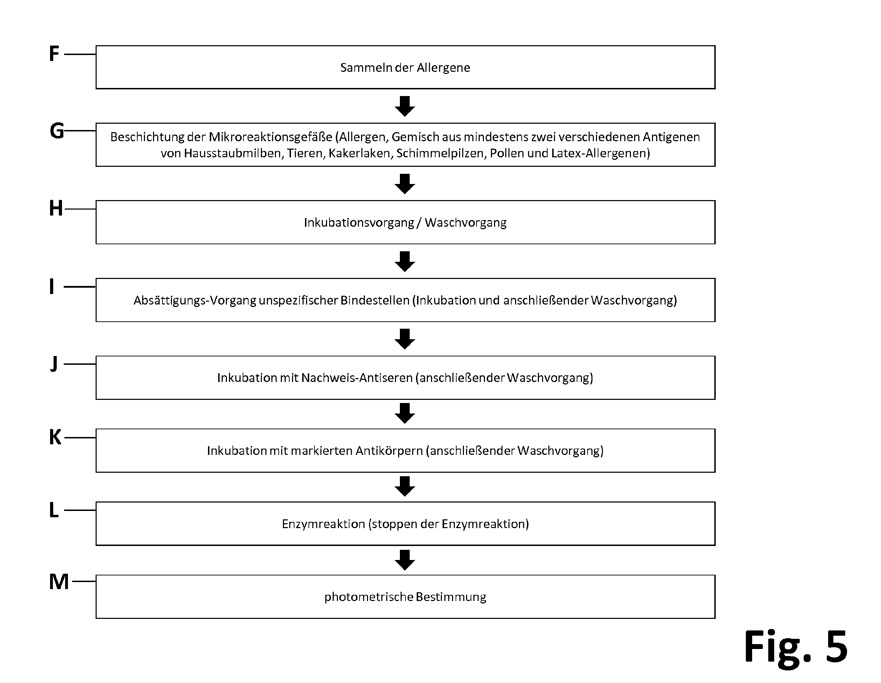

Die Figur 5 zeigt ein Diagramm über den Ablauf des immunologischen Verfahrens (ELISA) zum Nachweis der biologischen Substanzen. Nach dem Sammeln der biologischen Substanzen aus der Luft 5 (F) wird den gesammelten Antigenen in den Mikroreaktionsgefäßen, sowohl ein Gemisch aus mindestens zwei Antigenen als auch parallel der Antigenstandard (hier das Extraktgemisch aus z. B. Schimmelpilzen) (G) in Puffer hinzugefügt. Anschließend erfolgt eine Inkubation für mehrere, bevorzugt vier, Stunden bei ungefähr 10° bis 30°C, bevorzugt ungefähr 20 °C (H). Unspezifische Bindungsstellen werden mit einem Absättigungspuffer für mehrere Minuten, bevorzugt für 60, bei ungefähr 10° bis 30°C, bevorzugt ungefähr 20 °C, (I) blockiert. Es erfolgt ein anschließender mehrmaliger, bevorzugt dreimaliger, Waschvorgang mit Waschpuffer. Anschließend erfolgt die Inkubationen mit dem entsprechenden Immunserum aus einem immunisierten Säugetier, bevorzugt einem Kaninchen, für mehrere Stunden, bevorzugt über Nacht, und einem enzymmarkierten Zweitantikörper, bevorzugt Peroxidase markierten Antikörper, bevorzugt einem IgG-Antikörper, besonders bevorzugt einem IgG-Antikörper aus Kaninchen (K), für mehrere Minuten, bevorzugt 60, bei ungefähr 10° bis 30°C, bevorzugt bei 20°C (J). Die nach jedem Inkubationsschritt vorhandenen nicht gebundenen Reagenzien werden jeweils in einem mehrmaligen, bevorzugt dreimaligen, Waschschritt entfernt (J). Anschließend wird die Enzymreaktion mittels des Substratpuffers gestartet, die nach ungefähr 1 bis 30 Minuten, bevorzugt nach ungefähr 8 bis 10 Minuten, gestoppt wird, bevorzugt durch Schwefelsäurelösung (L). Die Messung erfolgt photometrisch bei 400 bis 500 nm, bevorzugt bei 405 oder bei 492 nm, Wellenlänge (M). Die gebundenen bzw. gesammelten Antigene werden anhand der mitgeführten Standardkurve durch eine PCgestützte Software quantifiziert. Das hier beschriebene Verfahren kann für Schimmelpilze und/oder Milben und/oder Tiere und/oder Kakerlaken und/oder Pollen und/oder Gummi, insbesondere Latex verwendet werden.

### Bezugszeichenliste

- 1: Probenträger, Mikroreaktionsgefäßstreifen
- 2: Mikroreaktionsgefäß
- 4: Kontrollmikroreaktionsgefäß
- 5: Luftstrom
- 6: Sammelvorrichtung
- 7: Gehäuse der Sammelvorrichtung
- 9: Beschichtung

## Patentansprüche

1. Verfahren zur Untersuchung von Luft (5) auf das Vorhandensein von biologischen Substanzen, wobei
eine Sammelvorrichtung (6) mit mindestens einem Probenträger (1) vorgesehen ist, die Luft (5) durch die Sammelvorrichtung (6) geleitet wird und somit eine definierte Zeit lang ein Sammelvorgang durchgeführt wird, bei dem am Probenträger (1) die biologischen Substanzen gesammelt werden,
und anschließend die biologischen Substanzen durch mindestens ein immunologisches Nachweisverfahren bestimmt werden,
**dadurch gekennzeichnet,**
**dass** nach dem Sammelvorgang der Probenträger (1) aus der Sammelvorrichtung (6) entnommen wird und in dem Probenträger (1) das immunologische Nachweisverfahren durchgeführt wird.

2. Verfahren nach Anspruch 1,
**dadurch gekennzeichnet,**
**dass** die biologischen Substanzen Proteine, bevorzugt Antigene sind, insbesondere dass es sich um Antigene von Schimmelpilzen und/oder Milben und/oder Tieren und/oder Kakerlaken und/oder Pollen und/oder Gummi, insbesondere Latex handelt.

3. Verfahren nach Anspruch 1 oder 2,
**dadurch gekennzeichnet,**
**dass** der Nachweis der biologischen Substanzen mittels einer immunologischen Reaktion, insbesondere mittels Antikörpern erfolgt,
und/oder dass der Sammelvorgang in einem Innenraum stattfindet, wobei eine Messung, insbesondere eine Referenzmessung, außerhalb geschlossener Räume stattfindet.

4. Verfahren nach einem der vorhergehenden Ansprüche,
**dadurch gekennzeichnet,**
**dass** bei dem Sammelvorgang biologische Substanzen aus mindestens 100 I, bevorzugt aus mindestens 1000 I und besonders bevorzugt aus mindestens 2000 I Luft (5) gesammelt werden,
und/oder dass bei dem Sammelvorgang mindestens 50 I/min und bevorzugt mindestens 100 I/min Luft (5) durch die Sammelvorrichtung (6) geleitet werden.

5. Verfahren nach einem der vorhergehenden Ansprüche,
**dadurch gekennzeichnet,**
**dass** die Dauer des Sammelvorgangs weniger als ungefähr 4 Stunden, bevorzugt weniger als ungefähr 2 Stunden und besonders bevorzugt weniger als ungefähr 1 Stunde beträgt,
und/oder dass der Sammelvorgang filterlos, insbesondere direkt in dem Probenträger (1), durchgeführt wird,
und/oder dass der Probenträger (1) medienundurchlässig, insbesondere undurchlässig für Luft (5) und die darin enthaltenen biologischen Substanzen ist.

6. Verfahren nach einem der vorhergehenden Ansprüche,
**dadurch gekennzeichnet,**
**dass** der Probenträger (1) Mikroreaktionsgefäße (2) aufweist, insbesondere, dass die Mikroreaktionsgefäße (2) nebeneinander angeordnet sind und einen Streifen (1) bilden.

7. Verfahren nach einem der vorhergehenden Ansprüche,
**dadurch gekennzeichnet,**
**dass** das immunologische Nachweisverfahren eine Kontrolle umfasst, die eine Aussage über die Richtigkeit des Sammel- und Nachweisverfahrens gibt,
und/oder dass für die Kontrolle mindestens ein zusätzliches Mikroreaktionsgefäß (2) vorgesehen ist, insbesondere dass die Kontrolle eine Negativkontrolle ist,
und/oder dass in das zusätzliche Mikroreaktionsgefäß (2) nahezu keine Luft (5), die während des Sammelvorgangs in die Sammelvorrichtung (6) geleitet wird, strömt.

8. Verfahren nach einem der vorhergehenden Ansprüche,
**dadurch gekennzeichnet,**
**dass** der Nachweis der biologischen Substanzen über eine enzymatische Reaktion, insbesondere einer enzymatischen Farbreaktion, erfolgt.

9. Sammelvorrichtung (6) zum Sammeln von biologischen Substanzen in Luft (5), zur Untersuchung von biologischen Substanzen, mit
einem Gehäuse (7), in dem mindestens ein Probenträger (1) angeordnet ist, wobei durch das Gehäuse (7) Luft (5) leitbar ist, wodurch
biologische Substanzen am Probenträger (1) in einem Sammelvorgang sammelbar sind,
**dadurch gekennzeichnet,**
**dass** der Probenträger (1) gefäßartig mit einer definierten Bindungskapazität derart ausgestaltet ist, dass beim Sammelvorgang biologische Substanzen zuverlässig verbleiben, insbesondere dass der Probenträger (1) Mikroreaktionsgefäße (2) aufweist, insbesondere, dass die Mikroreaktionsgefäße (2) nebeneinander angeordnet sind und einen Streifen (1) bilden.

10. Sammelvorrichtung (6) nach Anspruch 9,
**dadurch gekennzeichnet,**
**dass** die Mikroreaktionsgefäße (2) aus Kunststoff, bevorzugt aus Polystyrol, gefertigt sind,
und/oder dass die Mikroreaktionsgefäße (2) Deckel aufweisen, um diese zu verschließen, wobei die Deckel aus Kunststoff, bevorzugt aus Polypropylen, gefertigt sind.

11. Sammelvorrichtung (6) nach Anspruch 9 und 10,
**dadurch gekennzeichnet,**
**dass** die Mikroreaktionsgefäße (2) eine hohe Bindungskapazität aufweisen, so dass eine hohe Haftungseigenschaft der biologischen Substanzen hervorgerufen wird
und/oder dass die Mikroreaktionsgefäße (2) eine Beschichtung (9) aufweisen, die bevorzugt aus Glutaraldehyd und/oder einem spezifischen Antikörper ausgebildet ist, wodurch eine erhöhte Bindungskapazität geschaffen wird.

12. Immunologisches Verfahren, insbesondere ELISA, zum Nachweis von Antigenen aus der Luft, umfassend
einen Probenträger (1) aus Mikroreaktionsgefäßen (2), in welchem die Antigene enthalten sind,
**dadurch gekennzeichnet,**
**dass** zur Erkennung der gesammelten Antigene ein Gemisch aus mindestens zwei Antikörpern zwei verschiedener Arten eingesetzt wird.

13. Immunologisches Verfahren nach Anspruch 12,
**dadurch gekennzeichnet,**
**dass** das immunologische Nachweisverfahren Proteine, insbesondere Antigene von Milben und/oder Tieren und/oder Kakerlaken und/oder Schimmelpilzen und/oder Pollen und/oder Gummi, insbesondere Latex nachweist.

14. Immunologisches Verfahren nach Anspruch 12 oder 13,
**dadurch gekennzeichnet,**
**dass** das Gemisch aus Antikörpern zur Erkennung der gesammelten Antigene ein Serum ist,
und/oder dass zur Erkennung der gesammelten Antigene ein Serum aus einem immunisierten Säugetier, bevorzugt aus einem immunisierten Nagetier und besonders bevorzugt aus einem immunisierten Kaninchen eingesetzt wird.

15. Immunologisches Verfahren nach Anspruch 14,
**dadurch gekennzeichnet,**
**dass** die Antikörper zur Erkennung der gesammelten Antigene, bevorzugt monoklonale und besonders bevorzugt polyklonale Antikörper sind.

16. Immunologisches Verfahren nach Anspruch 14 oder 15,
**dadurch gekennzeichnet,**
**dass** zur Herstellung des Serums ein Gemisch aus mindestens zwei verschiedenen Antigenen von Milben und/oder Tieren und/oder Kakerlaken und/oder Schimmelpilzen und/oder Pollen und/oder Gummi, insbesondere Latex eingesetzt wird, wobei das Gemisch bevorzugt aus mindestens vier verschiedenen Antigenen und besonders bevorzugt aus mindestens 100 verschiedenen Antigenen eingesetzt wird.

17. Immunologisches Verfahren nach Anspruch 12 bis 16,
**dadurch gekennzeichnet,**
**dass** ein Gemisch aus Antikörpern mindestens die Arten Aspergillus penicilloides und/oder Cladosporium cladosporioides umfasst.

18. Immunologisches Verfahren nach Anspruch 12 bis 17,
**dadurch gekennzeichnet,**
**dass** das Gemisch aus Antikörpern eine Kombination der Antigene von Aspergillus penicilloides und Aspergillus versicolor und/oder ein Gemisch aus Aspergillus penicilloides und Penicillium chrysogenum und/oder aus Cladosporium cladosporioides und Aspergillus versicolor und/oder aus Cladosporium cladosporioides und Penicillium chrysogenum und/oder aus Aspergillus versicolor und Penicillium chrysogenum und/oder aus Aspergillus penicilloides und Cladosporium cladosporioides und Aspergillus versicolor und/oder aus Aspergillus penicilloides und Cladosporium cladosporioides und Penicillium chrysogenum und/oder aus Aspergillus penicilloides und Aspergillus versicolor und Penicillium chrysogenum und/oder aus Cladosporium cladosporioides und Aspergillus versicolor und Penicillium chrysogenum und/oder aus Cladosporium cladosporioides und Penicillium chrysogenum und Aspergillus penicilloides und/oder aus Aspergillus penicilloides und Cladosporium cladosporioides und Aspergillus versicolor und Penicillium chrysogenum umfasst.
